# EUROPÄISCHE PATENTSCHRIFT

(11) **EP 4 440 511 B1**
(45) Veröffentlichungstag und Bekanntmachung des Hinweises auf die Patenterteilung: **25.11.2026**
(21) Anmeldenummer: 22808950.4
(22) Anmeldetag: 10.11.2022
(51) Int. Cl.: A61F 5/02

(54) **RÜCKENORTHESEVORRICHTUNG INSBESONDERE ZUR REKLINATION SOWIE VERWENDUNG**
BACK BRACE DEVICE, IN PARTICULAR FOR RECLINING, AND USE THEREOF
DISPOSITIF D'ORTHÈSE DORSALE, EN PARTICULIER POUR L'INCLINAISON, ET SON UTILISATION

(30) Priorität: 30.11.2021 DE 102021131494
(43) Veröffentlichungstag der Anmeldung: 09.10.2024
(73) Patentinhaber: Spektramed GmbH, 41844 Wegberg (DE)
(72) Erfinder: SCHREINEMACHER, Maximilian, 41844 Wegberg (DE)
(74) Vertreter: Tanner, Lukas
(86) Internationale Anmeldenummer: PCT/DE2022/100836
(87) Internationale Veröffentlichungsnummer: WO 2023/098936

(56) Entgegenhaltungen:
- DE-U1- 202021 106 539
- US-A1- 2011 152 737
- US-A1- 2014 018 715
- US-A1- 2015 133 842

## Beschreibung

### TECHNISCHES GEBIET

Die vorliegende Erfindung betrifft eine Rückenorthese bzw. Rückenorthesevorrichtung, insbesondere zur Reklination des Rückens einer Person, aufweisend eine Rückenschiene und wenigstens einen Schultergurt und für die Schultern der Person jeweils wenigstens einen Zugmittelabschnitt, wobei diese Zugmittelabschnitte an die Rückenschiene koppelbar sind und bedienbar sind. Ferner betrifft die vorliegende Erfindung auch die Verwendung einer Einheit zum Bedienen der wenigstens zwei auf die Schultern einer die Rückenorthese tragenden Person wirkenden Zugmittelabschnitte in einer solchen Rückenorthesevorrichtung. Insbesondere betrifft die Erfindung eine Vorrichtung mit der Funktionalität einer Rückenorthese, insbesondere eingerichtet zur Reklination, und Verwendungen jeweils gemäß Merkmalen des entsprechenden unabhängigen Anspruchs.

### HINTERGRUND DER ERFINDUNG

Rückenorthesen werden z.B. zur Reklination des Oberkörpers verwendet. Derartige Orthesen weisen üblicherweise zwei Schultergute auf, mittels welchen die gewünschte Kraft insbesondere auf die Schultern einer Person (oder eines sonstigen Individuums) ausgeübt werden kann. Die Schultergute sind mit einer Rückenschiene gekoppelt und werden z.B. ähnlich wie beim Aufsetzen eines Rucksack angelegt. Je nach Anwendungsart ist ein recht individuelles Einstellen der Orthese und auch einer dadurch auf die Person ausgeübten Kraft bzw. das damit einher gehende Ausmaß einer Haltungsänderung gewünscht bzw. aus medizinischer, insbesondere orthopädischer Sicht auch erforderlich. Je nach Verfassung der Person, insbesondere auch älterer Personen (z.B. eines auch anderweitig beeinträchtigten Patienten), und je nach Situation und gewünschter Anwendungsart kann das Anlegen oder Ablegen der Orthese und/oder das Einstellen der Orthese mit vergleichsweise großem Aufwand oder gar Unannehmlichkeiten für die involvierten Personen verbunden sein. In der Tat sind die Vorgänge Anlegen und Spannen (Kraftaufbringung auf die Schultern) in vielen Fällen täglich wiederkehrende Vorgänge, die möglichst einfach durchzuführen sein sollten. Beispielsweise auch ein wiederholtes Einstellen der Orthese sollte auf möglichst einfache Weise in möglichst gleichbleibender Stellung personenspezifisch und gegebenenfalls auch ganz ohne Mithilfe von Fachkräften sichergestellt werden können, insbesondere auch bei minimiertem Fehlerpotential. Es besteht demnach Interesse an einer verbesserten und erleichterten Ergonomie und Einstellmöglichkeit.

Bisher werden die Schultergurte üblicherweise über ein Gurtkreuz geführt und beidseitig am Körper eingestellt und fixiert. Beispielhaft kann in diese Zusammenhang die Veröffentlichung EP 3 407 842 B1 genannt werden, welche eine Anordnung der Schultergurte mit dorsalem zentralem Kreuzungspunkt beschreibt.

Ferner können die beiden folgenden Veröffentlichungen genannt werden: US 2011/0152737 A1 beschreibt eine Anordnung von Schultergurten, bei welcher die Schultergurtenden in einem gemeinsamen Punkt in Höhenrichtung verlagerbar sind; US 2010/0113995 A1 beschreibt eine Schulterplatte, über welche Zugmittel geführt sind.

Ergänzend kann auch die folgende Veröffentlichung genannt werden: US 2014/018715 A1 beschreibt eine Wirbelsäulenorthese mit einer Gurtanordnung, die an einem oberen Abschnitt eines Wirbelsäulenrahmens befestigt ist, wobei sich zwei Gurtsegmente zu einer verschiebbaren Gurtbefestigung erstrecken, welche zwei Klammern definiert, mittels welchen die Gurtsegmente umgelenkt werden. Die Gurtsegmentewerden nicht über die Rückenschiene geführt, sondern seitlich davon umgelenkt, und die Gurtsegmente werden einzeln bedient, insbesondere indem ein jeweiliges Basisteil des entsprechenden Gurtsegments zur Befestigung am entsprechenden Gürtelsegment gehandhabt wird.

Ergänzend kann auch die folgende Veröffentlichung genannt werden: US 2015/133842 A1 beschreibt eine Gurtführung, bei welcher ein jeweiliger Schultergurt nach oben gegen eine Rückenschiene gezogen werden kann und dabei durch einen mittig im Thorax-Bereich lateral angreifenden Gurt dorsal gehalten wird und weiter nach caudal/ventral geführt wird. Das sich ergebende Zugkraftprofil erstreckt sich im Wesentlichen caudal-cranial im ventralen Bereich.

Ausgehend vom Stand der Technik ist ein Optimierungsbedarf hinsichtlich des Aufbaus und der Bedienbarkeit von Rückenorthesen zu spüren. Nicht zuletzt besteht insbesondere hinsichtlich möglichst kleinem Kraftaufwand und möglichst fehlerfreier Anwendung auch Interesse an einem besonders einfachen und reibungsarmen Mechanismus zum personenindividuellen Einstellen der Orthese.

### ZUSAMMENFASSUNG DER ERFINDUNG

Aufgabe ist, eine Rückenorthese bereitzustellen, mittels welcher die Handhabung an der tragenden Person (bzw. Individuum) vereinfacht werden kann, insbesondere hinsichtlich Spannen und Fixieren. Auch ist es Aufgabe, die Rückenorthese derart auszuführen bzw. auszugestalten, dass die Handhabung sicher und intuitiv und wenig fehleranfällig erfolgen kann, insbesondere auch bezüglich eines An-/Ablegevorgangs am Oberkörper (Handhabung durch die tragende Person oder durch eine helfende weitere Person). Auch ist es Aufgabe, die Rückenorthese derart auszuführen bzw. auszugestalten, dass eine Wirkung der Orthese hinsichtlich Reklination des Oberkörpers der tragenden Person auf besonders einfache Weise eingestellt und wahlweise auch justiert/dosiert werden kann.

Diese Aufgabe wird durch eine Rückenorthesevorrichtung gemäß Anspruch 1 sowie durch Verwendungen gemäß dem nebengeordneten Verwendungsanspruch gelöst. Vorteilhafte Weiterbildungen der Erfindung werden in den jeweiligen Unteransprüchen erläutert. Die Merkmale der im Folgenden beschriebenen Ausführungsbeispiele sind miteinander kombinierbar, sofern dies nicht explizit verneint ist.

Bereitgestellt wird eine Rückenorthesevorrichtung, insbesondere zur Reklination des Rückens einer Person, aufweisend eine Rückenschiene und wenigstens einen an der Rückenschiene befestigten Schultergurt und für die rechte und linke Schulter der Person jeweils wenigstens einen über den oder im Schultergurt geführten oder am Schultergurt befestigten Zugmittelabschnitt, wobei diese Zugmittelabschnitte an die Rückenschiene gekoppelt/koppelbar sind und von der Person oder einer weiteren, assistierenden Person zur Zugkraftaufbringung und zum Einstellen der vom Schultergurt ausgeübten Kraft bedienbar sind;
Erfindungsgemäß wird vorgeschlagen, dass die Rückenorthesevorrichtung eine an die Rückenschiene gekoppelte/koppelbare Zugkraftzentriereinheit aufweist, über/durch welche die auf beide Schultern wirkenden Zugmittelabschnitte zentral geführt sind und zusammen bedienbar sind. Dies erleichtert nicht nur das Aufbringen der Zugkraft, vielmehr kann die Zugkraft dank der Zentriereinheit auch vergleichsweise symmetrisch auf beide Schultern aufgebracht werden; insbesondere kann ein Risiko einer asymmetrischen Zugkraftverteilung minimiert werden. Die Orthese kann zudem auf sehr ergonomische und intuitive Weise gehandhabt werden, wobei eine die Orthese tragende Person (sofern sie keine Unterstützung durch eine helfende Person erhält) wahlweise die linke oder rechte Hand nutzen kann, um das/die Zugmittel einzustellen. Hierdurch ergibt sich nicht zuletzt auch für einseitig verletzte oder beeinträchtigte Personen eine leichtere Anwendbarkeit, möglicherweise ganz ohne assistierende Personen. Auch eine gewünschte hohe Akzeptanz seitens der tragenden Person für die Orthese als Hilfsmittel kann basierend auf der vorliegenden Erfindung leichter sichergestellt werden.

Die Erfindung beruht auch auf der Erkenntnis, dass beide Schultergurte gemeinsam über ein (einziges) Zugmittel bedient werden können (nur eine Systemschnur). Hierdurch kann auch der Vorteil erzielt werden, dass jeder Schultergurtabschnitt (rechte und linke Schulter) vergleichsweise weit geöffnet bzw. freigegeben werden kann, und auch bei vergleichsweise geringerem Kraftaufwand durch eine einzelne/einzige auf beide Seiten/Schultern wirkenden Zugbewegung (Ziehen) wieder geschlossen werden kann. Insofern betrifft die Erfindung auch ein verbessertes Verschlusssystem.

Als "Reklination" ist hier insbesondere allgemein das (dorsale) Zurückstellen bzw. Rückwärtsneigen des Oberkörpers eines Individuums, insbesondere eines Menschen zu verstehen, beispielsweise im Zusammenhang mit medizinischen, insbesondere orthopädischen Maßnahmen, z.B. im Zusammenhang mit dem Zurückbiegen/Zurückstellen bei einer so genannten Reklinationsbehandlung. Auch insofern bezieht sich die hier beschriebene Vorrichtung im Wesentlichen auf eine Einwirkung auf den Oberkörper; wahlweise kann die Vorrichtung bzw. ein entsprechendes System auch einen Hüftgurt umfassen oder zumindest damit zusammenwirken. Der Hüftgurt ist beispielsweise ebenfalls an die Rückenschiene kuppelbar bzw. daran befestigt. Wahlweise kann an der Rückenschiene auch ein anderer Gurt als ein Hüftgurt vorgesehen sein, insbesondere ein Gurt, welcher den Oberkörper der Person in einer Transversalebene umgibt, und an welchem das Zugmittel fixiert werden kann, z.B. mittels Klettverschluss.

Die hier genutzten Richtungsangaben "rechts" und "links" beziehen sich auf eine Blickrichtung auf den Rücken der Person, also von hinten in Sagittalebene in anteriorer/ventraler Richtung; insofern entspricht die hier als "rechte Schulter" bezeichnete Schulter der tatsächlich rechten Schulter der jeweiligen Person; insofern entspricht auch der hier als "rechter Zugmittelabschnitt" bezeichnete Zugmittelabschnitt dem bei bestimmungsgemäßer Anwendung der Vorrichtung an der rechten Schulter oder an der rechten Rückenhälfte anliegenden Zugmittelabschnitt. Die hier genutzten anatomischen Richtungsangaben (z.B. caudal, ventral) beziehen sich jeweils auf eine bestimmungsgemäße Anordnung der Orthese bzw. des Adapters an der jeweiligen Person.

Als "Zugkraftzentriereinheit" ist insbesondere eine Einheit zum Zentrieren der beidseitig auf beide Schultern bzw. Körperseiten (rechts, links) ausgeübten Zugkräfte auf ein vergleichsweise kleines von der Einheit definiertes Gebiet zu verstehen, insbesondere auf ein zumindest annähernd punktförmiges oder nur z.B. fingernagelgroßes Zugkraftzentrum; insofern kann der Begriff "Zugkraftzentriereinheit" in einer der möglichen Auslegungen auch im Sinne von Zugmittelzentriereinheit verwendet/verstanden werden. Bevorzugt ist die Zugkraftzentriereinheit zentral in der Sagittalebene angeordnet, insbesondere bei entlang der Rückenschiene einstellbarer Höhenposition. Die Zentriereinheit kann insbesondere bei spezifischer Bezugnahme auf eine Orthese für die Reklination des Rückens bzw. der Schultern auch als Reklinationsadapter bezeichnet werden. Die Zugkraftzentriereinheit umfasst jedenfalls die Funktionalität des Zusammenführens von zwei Zugmittelabschnitten derart, dass eine unilaterale Bedienung erfolgen kann. Wahlweise kann die Zugkraftzentriereinheit auch eine Kraftverstärkungsfunktion erfüllen/bereitstellen, insbesondere mittels einer flaschenzugartigen Führung/Umlenkung des Zugmittels.

Personifizierte Begriffe, soweit sie hier nicht im Neutrum formuliert sind, können im Rahmen der vorliegenden Offenbarung alle Geschlechter betreffen. Etwaige hier verwendete englischsprachige Ausdrücke oder Abkürzungen sind jeweils branchenübliche Fachausdrücke und sind dem Fachmann in englischer Sprache geläufig. Etwaige dazu synonym verwendete/verwendbare deutschsprachige Begriffe können hier der Vollständigkeit halber in (Klammern) angegeben werden, oder vice versa.

Mit anderen Worten stellt die Erfindung auch ein Orthesen-Verschlusssystem bereit, welches eine Gurt- oder Zugmittel-Adapterfunktion (kinematische Kopplung) zum zentralen Bedienen von wenigstens zwei Schultergurtabschnitten mittels eines Zugmittels liefert (insbesondere nur eine einzige die zugkraftausübenden Komponenten miteinander verbindende Schnur), wobei die Adapterfunktion wahlweise auf eine größere Anzahl von Gurtabschnitten oder eine andere Art von Gurten übertragbar ist, falls für bestimmte Orthesen-Anwendungen gewünscht. Die Kraftübertragung erfolgt beispielsweise über mehrere Umlenkrollen (insbesondere aus Polymer-Material, z.B. Polytetrafluorethylen PTFE), insbesondere fünf Umlenkrollen. Ferner ist im jeweiligen zu bedienenden Schultergurtabschnitt bevorzugt wenigstens eine Umlenkrolle vorgesehen, insbesondere zwecks Minimierung einer Reibung bei Realisierung einer Flaschenzuganordnung. Die komplette Schnurführung (Zugmittelanordnung) beginnt und endet bevorzugt in einem händisch freibaren Zug- bzw. Bedienelement (Bedieneinheit) und bildet somit ein geschlossenes System. Das komplette System ist demnach bevorzugt derart ausgelegt, dass basierend auf dem Prinzip eines Flaschenzugs die vom Schultergurt ausgehenden Kräfte über einen zentralen Kraftangriffspunkt auf die Rückenschiene übertragen werden, wobei beim Anlegen und Spannen der Orthese die aufzubringende Zugkraft an nur einer Seite des Körpers der Person aufgebracht werden muss; die dafür vorgesehene Bedieneinheit kann dabei auch unilateral, z.B. links im Bereich der Hüfte oder des Bauchs der Person, fixiert werden. Somit ist ein Kraftfluss bzw. Kraftschluss über ein einziges Zugelement auf beide Reklinations-Schultergurtabschnitte möglich. Dabei sind die Zentriereinheit (bzw. der kinematische Adapter) und die Gurtaufnahmen (freie Enden der jeweiligen Schultergurtabschnitte) bevorzugt derart geometrisch zueinander korrespondierend ausgestaltet, dass die Gurtaufnahmen im/am Adapter "andocken" können (insbesondere formschlüssig, zumindest stirnseitig anliegend) und wahlweise auch in dieser Endstellung verriegelt werden können. Die Zentriereinheit kann beispielsweise mittels zwei Schrauben an der Rückenschiene befestigt sein, z.B. an zwei T-Nutensteinen aus Aluminium. Bevorzugt sind die Nutensteine oder dergleichen Befestigungsmittel in einer Nut innerhalb der Schiene angeordnet, was in Hinblick auf eine Höhenverstellbarkeit des Kraftangriffspunktes an der Schiene von Vorteil ist. Die Zug-/Bedieneinheit kann beispielsweise mittels einen Klettverschlusses an einem entsprechenden Flächenabschnitt an der Orthese befestigt bzw. positioniert werden und kann dabei auch in Hinblick auf gute Ergonomie breitflächig insbesondere in der Länge eingestellt/positioniert werden (Zuführung von mehr oder weniger Schnurlänge, insbesondere auch zwecks Größenanpassung).

Beispielsweise ist die Zentriereinheit oder der Adapter, oder zumindest deren/dessen außenliegende Bestandteile (insbesondere Außenflächen, Gehäuseabschnitte), einstückig integral aus einem einzelnen Bauteil bereitgestellt, z.B. aus Kunststoff. Wahlweise können auch zwei Teile vorgesehen sein, z.B. ein Oberteil und ein Unterteil (insbesondere jeweils in Ausgestaltung als Schale oder Gehäuseseite); eine zweiteilige Ausgestaltung hat z.B. hinsichtlich Integration und Anordnung der Umlenkmittel Vorteile. Zur Herstellung können, je nach gewünschter Stückzahl, beispielsweise 3D-Druck-Verfahren oder Spritzguss herangezogen werden. Bezüglich der Materialauswahl ist der Fachmann frei hinsichtlich z.B. metallischer Materialien oder Kunststoffmaterialien.

Gemäß einem Ausführungsbeispiel sind die auf beide Schultern wirkenden Zugmittelabschnitte durch ein beidseitig wirkendes umlaufendes Zugmittel bereitgestellt, welches bevorzugt unilateral an nur einer Seite der Person geführt/führbar und bedienbar ist, also auf nur einer Seite des Oberkörpers der Person bedient werden muss. Dies begünstigt die Handhabung noch weiter, und kann z.B. auch ein einhändiges Bedienen (Spannen, Verschließen) ermöglichen. Indem die Zugkraft auf beide Schultern über ein einziges Zugmittel eingestellt wird, insbesondere mittels nur einer einzigen Bedieneinheit, kann ein korrekter Sitz der Orthese am Körper vergleichsweise unproblematisch eingestellt werden. Insbesondere muss nur eine einzige Einstellposition beachtet werden (also eben nicht wie bisher üblich wenigstens zwei Positionen für die rechte und linke Körperseite).

Gemäß einem Ausführungsbeispiel ist das beidseitig wirkende umlaufende Zugmittel aus der Zugkraftzentriereinheit herausgeführt, insbesondere über zwei Umlenkrollen, insbesondere mit zwei Strängen, und in einem lateralen und/oder ventralen Bereich der Person handhabbar und bevorzugt auch fixierbar, beispielsweise an einem lateral und/oder ventral verlaufenden Klettverschlussabschnitt der Rückenorthesevorrichtung oder eines zusätzlichen Gurts, insbesondere Hüftgurts. Anders ausgedrückt: Die Zugmittelabschnitte bzw. das Zugmittel verläuft/verlaufen von den Schultergurten über die Zentriereinheit und können von dort weitergeleitet bzw. zur Bedienung freigegeben werden. Dies ermöglicht auch, die Interaktion der tragenden Person mit dem Zugmittel zentral über die Zentriereinheit zu führen und dabei die eingeleiteten Kräfte bevorzugt auch direkt in die Rückenschiene zu leiten. Insofern kann auch eine sehr stabile Anordnung mit direktem Kraftfluss und wenig Reibungsverlusten bereitgestellt werden. Bevorzugt ist die Zentriereinheit unmittelbar an die Rückenschiene gekuppelt bzw. direkt mit dieser verbunden, z.B. daran angeschraubt. Mittels einer Schraubverbindung umfassend eine oder mehrere Schrauben kann dabei auch ein gutes Sicherheitsniveau sichergestellt werden.

Gemäß einem Ausführungsbeispiel ist das Zugmittel in der Zugkraftzentriereinheit um ein Zugkraftzentrum herum geführt, wobei die Zugkraftzentriereinheit bevorzugt im Bereich des Zugkraftzentrums an die Rückenschiene gekoppelt ist, z.B. mittels Schraubverbindung. Dies liefert auch eine für den Kraftfluss besonders vorteilhafte Anordnung und auch eine vorteilhafte Kraftkopplung mit der Rückenschiene, insbesondere ohne dass eine Person irgendeine Kupplung oder irgendeinen Befestigungsmechanismus an der Rückenschiene bedienen muss. Anders ausgedrückt: Die in vielen Fällen auch sicherheitsrelevante Verbindung zwischen Zugmittel (und damit auch Schultergurt) und Rückenschiene kann erfindungsgemäß ständig verbunden (ungelöst) bleiben (Minimierung eines Risikos hinsichtlich technischem Versagen oder hinsichtlich Fehlbedienung). Beispielsweise sind alle Umlenkrollen der Zentriereinheit insbesondere in sternförmiger Anordnung um das Zugkraftzentrum herum angeordnet, und das Zugmittel wirkt mit einem lateralen Druck nach außen auf die Achse der jeweiligen Umlenkrolle, so dass Zugkraftzentriereinheit Zugkräfte aufnimmt, die (in einer Frontalebene gesehen) radial nach außen gerichtet sind.

Gemäß einem Ausführungsbeispiel weist die Zugkraftzentriereinheit eine Mehrzahl von Umlenkmitteln, insbesondere Rollen auf, beispielsweise vier oder fünf Umlenkmittel. Dies ermöglicht nicht zuletzt eine sichere und für die Stabilität der gesamten Anordnung vorteilhafte Vorgabe des Kraftflusses und der Führungsbahn der Zugmittelabschnitte. Insofern beruht die vorliegende Erfindung auch auf der Erkenntnis, dass es hinsichtlich Kraftflusspfad und Krafteinleitung vorteilhaft ist, die Systemschnur (Zugmittel) durch ein Rollensystem auf der Rückseite der Orthese zu führen. Beispielsweise sind die Umlenkrollen derart angeordnet, dass das Zugmittel in einer Transversalebene in einem Winkel von zumindest annähernd 45° lateral-caudal aus der Zentriereinheit heraus zu einer/zur Bedieneinheit (bzw. zu einer Hand oder Hüfte der die Orthese tragenden Person) geführt werden kann. Beispielsweise sind die Umlenkrollen derart angeordnet, dass ein/das auch über das jeweilige freie Ende des Schultergurts umlaufend geführte Zugmittel sternförmig um das Zugkraftzentrum geführt wird, wobei wahlweise ein Paar von Umlenkrollen vorgesehen ist, mittels welchem eine S-förmige Führungsbahn bereitgestellt wird, zum Zusammenführen von den beiden auf die rechte und linke Schulter einwirkenden Zugmittelabschnitten für das einseitige/unilaterale Bedienen. Dabei können zumindest lateral außenliegende Umlenkmittel (also solche die lateral beabstandet zum Mittelpunkt oder zur Sagittalebene angeordnet sind) bevorzugt in einer bezüglich einer/der Frontalebene (orthogonal zur Sagittalebene) geneigten Ausrichtung angeordnet sein (bei bestimmungsgemäßer Anordnung der Zugkraftzentriereinheit am Rücken der Person), insbesondere mit einem Neigungswinkel im Bereich von 3° bis 25°, insbesondere 10° bis 20°. Dies begünstigt nicht zuletzt auch eine ergonomische Anordnung der Zentriereinheit an der Rückenschiene, also sehr nahe an der Körperoberfläche, so dass eine sichere Zugmittelführung z.B. auch dann sichergestellt ist, wenn die Person einen stark durchgebogenen Rücken oder ein Hohlkreuz oder dergleichen aufweist.

Dabei kann die Zentriereinheit mittels des Zugmittels auch eine kinematische Kopplung zwischen den freien Enden der Schultergurte (insbesondere Verschlusszuglaschen) und der händisch bedienbaren Bedieneinheit zum Aufbringen der Zugkraft bereitstellen.

Gemäß einem Ausführungsbeispiel ist eine/die Mehrzahl von Umlenkmitteln der Zugkraftzentriereinheit in Flaschenzuganordnung zur Erhöhung der vom Zugmittel auf den Schultergurt ausgeübten Zugkraft vorgesehen, insbesondere mit zumindest einer Umlenkung zwischen einem/dem jeweiligen freien Ende des Schultergurts und dem/den korrespondierenden Umlenkmittel(n) der Zugkraftzentriereinheit, insbesondere mit zumindest einem Umlenkpunkt bzw. zumindest einer Umlenkachse am jeweiligen freien Ende des Schultergurts (bzw. am freien Ende des jeweiligen Schultergurtabschnitts). Dies kann den erforderlichen Kraftaufwand weiter minimieren, insbesondere derart stark minimieren, dass die über das Zugmittel von der Person aufzubringende Zugkraft für beide Schultern kleiner ist als bei herkömmlicher Gurtführung für nur eine Schulterseite. Die Übersetzung/Skalierung der Flaschenzuganordnung hinsichtlich Anzahl und Anordnung der Umlenkmittel (insbesondere Rollen) kann dabei auch je Anwendungsfall individuell optimiert/vordefiniert werden.

Gemäß einem Ausführungsbeispiel definiert die Zugkraftzentriereinheit für den jeweiligen Schultergurt eine Endstellung, insbesondere indem für jeden Schultergurt ein formstabiler Kupplungsabschnitt vorgesehen ist, welcher mittels des Zugmittels bzw. mittels der Zugmittelabschnitte formschlüssig in der Endstellung positionierbar ist. Eine derartig vordefinierte Endstellung (insbesondere geometrischer Anschlag) liefert auch den Vorteil, dass ein Überspannen des Schultergurts und damit eine zu große (Reklinations-)Kraft vermieden werden kann. Auch insofern kann ein Fehlerpotential bezüglich Fehlbedienung durch Laien weiter minimiert werden (technische Limitierung der der Reklinationswirkung). Wahlweise kann die Endstellung durch einen Rastmechanismus definiert sein. Dabei hat das vorliegende System auch den Vorteil, dass die Endstellung dabei für beide Schultern jedenfalls auf symmetrische Weise angefahren werden kann. Wahlweise kann über eine einstellbare Länge des wirksamen Zugmittels, z.B. Einstellmöglichkeit im Bedienelement, und/oder über eine Anpassung der Länge der Schultergurtabschnitte (z.B. mittels so genannter Y-Klett-Verbindungen), die individuelle Endstellung nachjustiert werden.

Optional ist der formstabile Kupplungsabschnitt eingerichtet zur Aufnahme und zur drehtoleranten Lagerung eines Drehtellers (insbesondere an der für die Umlenkrolle vorgesehenen Achse), welcher bevorzugt eine an den Adapter (Zentriereinheit) kuppelbare (bzw. damit interagierende) stirnseitige Flanke bereitstellt, insbesondere derart dass mittels des Drehtellers eine Drehwinkeltoleranz zwischen Adapter und jeweiligem Kupplungsabschnitt (bzw. rechtem und linkem Schultergurtabschnitt) implementierbar ist. Beispielsweise ist der Kupplungsabschnitt derart ausgestaltet, dass die stirnseitige Flanke wahlweise durch einen fest verbauten (integralen oder mehrteiligen) Abschnitt des Kupplungsabschnitts oder durch den drehwinkeltolerant gelagerten Drehteller bereitgestellt ist, so dass eine Individualisierung der Vorrichtung optional durch nachgerüstete Drehteller erfolgen kann (Zusatzoption ohne weitere konstruktive Abwandlungen).

Gemäß einem Ausführungsbeispiel ist der jeweilige Schultergurt in der Endstellung in einer durch die Zugkraftzentriereinheit (insbesondere in einer Frontalebene oder in einer Ausrichtung mit vordefiniertem Neigungswinkel relativ zur Frontalebene) vorgegebenen Position angeordnet, insbesondere indem der Schultergurt dort einrastet oder zumindest formschlüssig positioniert wird bzw. positionierbar ist. Hierdurch können auch eine gute Stabilität der Anordnung sowie ein guter Kraftfluss sichergestellt werden, und die korrekte Endstellung/Einstellung für die Reklinationswirkung kann derart intuitiv erkannt werden, dass auch eine Bedienung durch Laien möglich ist.

Gemäß einem Ausführungsbeispiel ist/sind das Zugmittel bzw. die Zugmittelabschnitte derart geführt, die Zugkraft unabhängig vom Erreichen der Endstellung auf die Schultern aufbringbar ist. Dies erleichtert nicht zuletzt auch das Bedienkonzept, wobei eine Flaschenzugwirkung bevorzugt in einem Abschnitt zwischen Zentriereinheit und dem freien Ende des jeweiligen Schultergurtabschnitts vorgesehen ist, insbesondere um das Erreichen der gewünschten/korrekten Endposition des Schultergurts zu vereinfachen.

Gemäß einem Ausführungsbeispiel weist die Zugkraftzentriereinheit an einer (ventralen) Unterseite Kupplungs- oder Befestigungsmittel für eine Befestigung an der Rückenschiene auf, wobei die Position eines/des Befestigungspunktes bevorzugt in Höhenrichtung, insbesondere bei bestimmungsgemäßer Verwendung der Rückenschiene in der Sagittalebene liegend, einstellbar durch die Rückenschiene vordefinierbar ist, insbesondere mittels einer Schiene oder Führung an der Rückenschiene. Dies begünstigt eine personenspezifische optimale Lage der Zentriereinheit und damit auch eines Kraftweiterleitungspunktes in die Rückenschiene, wobei die Höhenverstellung auch eine Größenanpassung bei unterschiedlichen Körpergrößen erleichtert. Beispielsweise sind zwei Befestigungs-/Schraubachsen vorgesehen, an welchen die Zentriereinheit an der Schiene oder Führung an der Rückenschiene feststellbar ist.

Gemäß einem Ausführungsbeispiel ist/sind das Zugmittel bzw. die Zugmittelabschnitte umlaufend und an einem freien von der Person bedienbaren Ende in einer Bedieneinheit zusammengeführt, welche ausgestaltet ist, beim An-/Ablegen der Schultergurte an der Zugkraftzentriereinheit zu blockieren, wobei das Zugmittel bzw. die Zugmittelabschnitte derart abgelängt ist/sind, dass der jeweilige Schultergurtabschnitt (rechts oder links, einer nach dem anderen) dank der kumulierten freien Zugweglänge beider Seiten cranial/lateral freilegbar ist. Dies erleichtert nicht nur das Aufbringen der Zugkraft und das symmetrische Einstellen der korrekten Länge der Gurte (insbesondere durch den Patienten selbst), sondern auch ein möglichst problemloses An-/ablegen der Orthese insbesondere auch für körperlich in der Bewegung eingeschränkte Patienten.

Die zuvor genannte Aufgabe wird auch gelöst durch ein modulares Orthesensystem mit einer zuvor weiter oben beschriebenen Rückenorthesevorrichtung, wobei das Orthesensystem ferner einen Hüftgurt umfasst, wobei ein/das beidseitig wirkende umlaufende Zugmittel der Rückenorthesevorrichtung bevorzugt in einem lateral und/oder ventral verlaufenden Klettverschlussabschnitt des Hüftgurts fixierbar ist. Hierdurch ergeben sich zuvor genannte Vorteile, insbesondere in Hinblick auf die Bereitstellung modularer Komponenten basierend auf möglichst nur wenigen Größenvariationen für eine Vielzahl unterschiedlicher Personen und variierender Körpergrößen. Der Hüftgurt ist beispielsweise in der Art eines Korsetts ausgestaltet. Der Hüftgurt ist beispielsweise an die Rückenschiene kuppelbar bzw. daran befestigt. Auf diese Weise kann auch ein modulares System bereitgestellt werden, bei welchem die einzelnen Komponenten in unterschiedlicher Zusammenstellung miteinander kombiniert werden können, z.B. zur Größenanpassung an bestimmte Körperformen/-größen von Patienten. Wahlweise kann die Rückenorthesevorrichtung auch Seitenteile aufweisen, welche derart ausgestaltet sind, dass alternativ oder zusätzlich ein Befestigungsabschnitt an wenigstens einem der Seitenteile für die Bedieneinheit vorgesehen ist, insbesondere eine Klettverschluss-Fläche im Hüft- und/oder Bauchbereich.

Die zuvor genannte Aufgabe wird auch gelöst durch Verwendung einer an eine Rückenschiene einer Rückenorthese gekoppelten/koppelbaren Zugkraftzentriereinheit zum zentralen Führen und gemeinsamen Bedienen von wenigstens zwei auf die Schultern einer die Rückenorthese tragenden Person wirkenden Zugmittelabschnitten, insbesondere im Zusammenhang mit einer Reklination des Rückens der Person, wobei die Zugmittelabschnitte jeweils derart an einen an der Rückenschiene befestigten Schultergurt gekoppelt sind/werden, dass die Person oder eine weitere, assistierende Person zur Zugkraftaufbringung und zum Einstellen der vom Schultergurt ausgeübten Kraft die Zugmittelabschnitte lediglich unilateral betätigen muss (insbesondere in einem lateralen und/oder ventralen Bereich der Person), insbesondere mittels einer die Zugmittelabschnitte zusammenführenden Bedieneinheit, wobei die Zugmittelabschnitte bevorzugt durch ein beidseitig wirkendes umlaufend durch die Zugkraftzentriereinheit geführtes und darin um ein Zugkraftzentrum in der Art eines Flaschenzugs umgelenktes Zugmittel bereitgestellt sind, insbesondere bei einer zuvor weiter oben beschriebenen Rückenorthesevorrichtung, insbesondere in einem modularen Orthesensystem zusammen mit einem Hüftgurt. Hierdurch lassen sich zuvor genannte Vorteile realisieren, insbesondere in Hinblick auf eine Minimierung des erforderlichen Kraftaufwandes und Minimierung eines Risikos bezüglich Bedien-/Einstellfehlern.

Zusammenfassung: Rückenorthesen insbesondere zur Reklination des Oberkörpers weisen Schultergurte und eine Rückenschiene auf. Jede Rückenorthese muss sehr individuell bezüglich der aufzuwendenden Kräfte eingestellt und auf die Größe der tragenden Person abgestimmt werden. Dem einfachen und exakten Einstellen der auf die Schultergurte zu übertragenden Zugkraft kommt dabei besondere Bedeutung zu. Bereitgestellt wird eine Rückenorthesevorrichtung mit wenigstens zwei auf die Schultergurte einwirkenden Zugmittelabschnitten, welche an die Rückenschiene gekoppelt/koppelbar sind und von der Person bedienbar sind. Erfindungsgemäß weist die Rückenorthesevorrichtung eine an die Rückenschiene gekoppelte/koppelbare Zugkraftzentriereinheit zum zentralen Führen und Bedienen der Zugmittelabschnitte auf. Hierdurch kann nicht nur die Ergonomie verbessert werden, sondern es wird auch ein sehr schnelles, unkompliziertes und exaktes Einstellen der gewünschten Wirkung der Schultergurte durch die tragende Person oder durch eine helfende weitere Person ermöglicht.

### KURZE BESCHREIBUNG DER FIGUREN

In den nachfolgenden Zeichnungsfiguren wird die Erfindung noch näher beschrieben, wobei für Bezugszeichen, die nicht explizit in einer jeweiligen Zeichnungsfigur beschrieben werden, auf die anderen Zeichnungsfiguren verwiesen wird. Es zeigen:
**Figuren 1A, 1B** in einer perspektivischen Seitenansicht und rückseitigen Ansicht eine Rückenorthesevorrichtung gemäß einem Ausführungsbeispiel;
**Figur 2** in einer leicht perspektivischen Draufsicht (in dorsaler Richtung) eine Detailansicht eines Adapters bzw. einer Zentriereinheit einer Rückenorthesevorrichtung gemäß Ausführungsbeispielen, wobei Umlenkmittel des Adapters zwecks besserem Verständnis freigelegt dargestellt sind;
**Figuren 3A, 3B, 3C** in zwei perspektivischen Seitenansichten und in einer stirnseitigen Seitenansicht (in caudaler Richtung) ein Gehäuse eines Adapters bzw. einer Zentriereinheit einer Rückenorthesevorrichtung gemäß Ausführungsbeispielen;
**Figuren 4A, 4B** in zwei perspektivischen Seitenansichten einen formstabilen Kupplungsabschnitt mit Anschlagskontur zum Bereitstellen einer Kupplung für das Zugmittel und zum Sicherstellen einer vordefinierbaren Endstellung in einer Rückenorthesevorrichtung gemäß Ausführungsbeispielen;
**Figuren 5A, 5B** in rückseitiger und in perspektivischer detaillierter Seitenansicht Komponenten einer Rückenorthesevorrichtung gemäß einem weiteren Ausführungsbeispiel;
**Figur 6** in perspektivischer Seitenansicht Komponenten eines Kupplungsabschnitts einer Rückenorthesevorrichtung gemäß einem weiteren Ausführungsbeispiel;

### DETAILLIERTE BESCHREIBUNG DER FIGUREN

Die Erfindung wird zunächst unter allgemeiner Bezugnahme auf alle Bezugsziffern und Figuren erläutert. Besonderheiten oder Einzelaspekte oder in der jeweiligen Figur gut sichtbare/darstellbare Aspekte der vorliegenden Erfindung werden individuell im Zusammenhang mit der jeweiligen Figur thematisiert.

Bereitgestellt wird eine Rückenorthesevorrichtung 10 mit einer Rückenschiene 11 mit rippenartigen Aussparungen 11.1 (insbesondere zum Sichergestellen der gewünschten Flexion bzw. Biegsamkeit der Schiene 11) und mit einem Schultergurt 13 mit einem rechten Schultergurtabschnitt 13a und einem linken Schultergurtabschnitt 13b. Am freien Ende 13.1 des jeweiligen Schultergurtabschnitts ist ein formstabiler Kupplungsabschnitt 13.3 vorgesehen (insbesondere mit Verschlusszuglasche). Wahlweise kann im/am Kupplungsabschnitt eine Längeneinstellmöglichkeit des jeweiligen Schultergurtabschnitts vorgesehen sein, wahlweise ist eine Längeneinstellung jedoch bewusst nur im Schulterbereich vorgesehen (insbesondere mittels so genannter Y-Klett-Verbindungen). Die beiden Schultergurtabschnitte 13a, 13b können mittels eines Zugmittels 15 bedient werden, welches durch eine zentral angeordnete Zentriereinheit 20 geführt ist, mittels welcher die Zugkraftwirkung einerseits zentriert, andererseits auch auf die Schultergurtabschnitte auf möglichst symmetrische Weise verteilt werden kann. Für jeden Schultergurtabschnitt ist ein entsprechender Zugmittelabschnitt 15a, 15b vorgesehen. Das Zugmittel 15 ist innerhalb der Zentriereinheit 20 über mehrere Umlenkrollen 21, 21b, 21c geführt, und auch am jeweiligen Kupplungsabschnitt bzw. freien Ende 13.1 ist bevorzugt eine Umlenkrolle 21a vorgesehen; somit kann mittels der Zentriereinheit 20 auch eine Kraftverstärkung basierend auf einer Flaschenzugkinematik bereitgestellt werden. Bevorzugt sind die beiden Zugmittelabschnitte 15a, 15b in einer Bedieneinheit 15.1 zusammengeführt und aneinander befestigt; die Zentriereinheit 20 kann demnach ein umlaufendes Zugmittel 15 bereitstellen, mittels welchem bei vergleichsweise geringem Kraftaufwand, unilateral aufgebracht, eine zentrale symmetrische Kraftverteilung und -übertragung auf beide Schultergurtabschnitte sichergestellt werden kann. Die Bedieneinheit 15.1 kann z.B. an einem Seitenteil 17 oder einem (Hüft-)Gurt 30 oder dergleichen lateraler Komponente der Orthese oder eines modularen Orthesensystems 100 befestigt werden, z.B. mittels Klettverschluss.

Eine Person 1 kann die Vorrichtung 10 insbesondere in der Art eines Rücksacks aufsetzen; dazu können die Schultergurtabschnitte 13a, 13b vergleichsweise weit freigegeben werden; die umlaufende Ausgestaltung des Zugmittels ermöglicht dabei ein weites Freilegen der jeweiligen Öffnung, durch welche der jeweilige Arm des Patienten hindurchgeführt werden muss; somit kann das Ab-/Anlegen der Orthese 10 auch ohne Entkoppeln von Gurten erleichtert und beschleunigt werden. Daraufhin können die Gurtabschnitte 13a, 13b zentral mittels der Bedieneinheit 15.1 in die gewünschte bzw. vordefinierte Position bis zur Zentriereinheit 20 gezogen werden, so dass sich eine vordefinierbare Rückstell-/Reklinationswirkung auf die Schultern der Person 1 einstellt. Die Zugkraftzentriereinheit kann demnach im Zusammenhang mit einer solchen Anwendung auch als Reklinationsadapter 20 bezeichnet werden. Die relativen Größenverhältnisse und die relative Anordnung eines durch die Zentriereinheit definierten Kraftzentrums C können mittels einer Höhenverstellung 11.3 (insbesondere Schiene oder Führung) an der Rückenschiene 11 eingestellt werden, falls erforderlich; dazu können unterschiedliche Arten von Kupplungs- oder Befestigungsmitteln 25 zwischen der Zugkraftzentriereinheit 20 und der Rückenschiene 11 vorgesehen sein, z.B. Schraubverbindungen und/oder Nutsteine oder dergleichen Kontermittel.

Zum verbesserten/erleichterten Einstellen der Orthese 10 weist die Zentriereinheit 20 bevorzugt einen Anschlag 23 für den Schultergurt 13 auf, mittels welchem eine Parkposition P bzw. eine Art formschlüssige Endstellung definiert werden kann. Dies hat auch den Vorteil, dass die Orthese z.B. nur einmal von einem Arzt, Arzthelfer, Therapeuten oder Orthopädiemechaniker für den jeweiligen Patienten eingestellt werden muss (insbesondere korrektes Einstellen der Länge der Schultergurtabschnitte), und daraufhin kann der Patient 1 mittels des Bedienelements 15.1 die Schultergurtabschnitte zentral und symmetrisch zuziehen und bis zur Endstellung positionieren; ein zu starkes Ziehen der Gurte kann demnach vermieden werden; es kann beispielsweise ausgeschlossen werden, dass der Patient versehentlich eine zu starke Reklinationswirkung einstellt.

Die folgenden (anatomische) Richtungsangaben können das Verständnis insbesondere hinsichtlich der Anwendung der Vorrichtung 10 erleichtern, wobei die zentral verlaufende Sagittalebene durch das Bezugszeichen (xy) markiert ist: anterior (ventral) a; posterior (dorsal) d; inferior (distal, caudal) u;

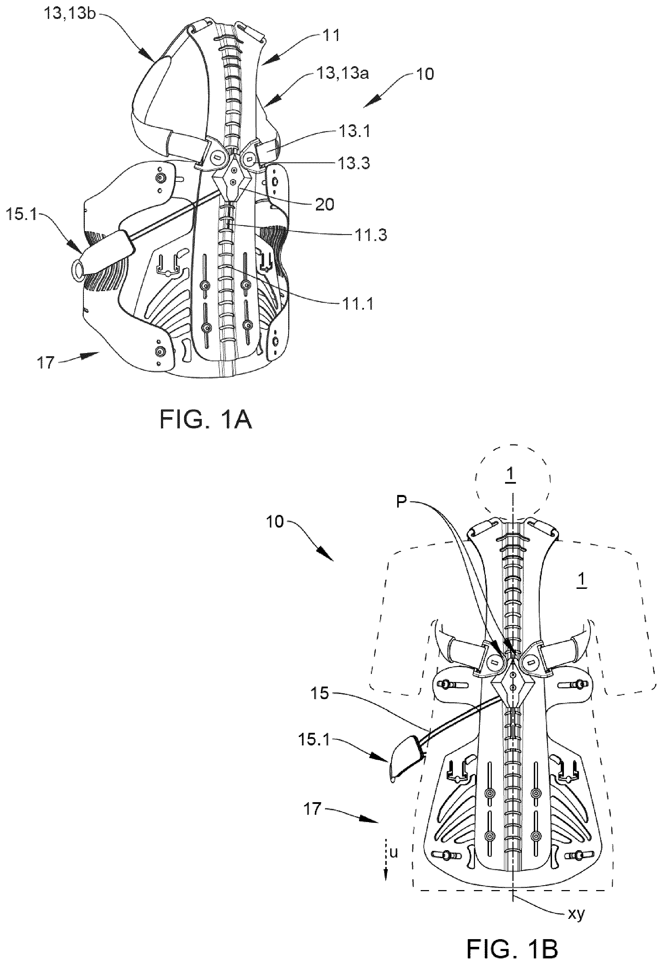

In **Fig. 1A** ist die relative Anordnung des Reklinationsadapters 20 an der Rückenschiene 11 erkennbar. Die Schultergurtabschnitte 13a, 13b sind in einer Anordnung in der Endstellung P gezeigt. Die Bedieneinheit 15.1 ist lateral links angeordnet.

In **Fig. 1B** ist eine Person 1 schematisch angedeutet. Es ist erkennbar, dass die Bedieneinheit 15.1 ergonomisch vorteilhaft im Bereich unter dem linken Oberarm der Person 1 verläuft. Die Person 1 kann die Bedieneinheit 15.1 ergonomisch vorteilhaft greifen und mit einer oder zwei Händen in ventraler Richtung nach vorne spannen und fixieren. Damit übt die Person 1 eine zentrale symmetrische Kraft auf beide Schultergurtabschnitte 13a, 13b aus, insbesondere so lange bis sich diese in der Endstellung P angedockt am Adapter 20 befinden. Dabei kann dank der im Folgenden näher erläuterten Umlenkmittel 21 auch eine Kraftverstärkung erfolgen. Aus den Figuren 1A, 1B geht auch hervor, dass reibungsintensive Gurtkreuze oder dergleichen aufeinander reibende Abschnitte erfindungsgemäß weitgehend vermieden werden können.

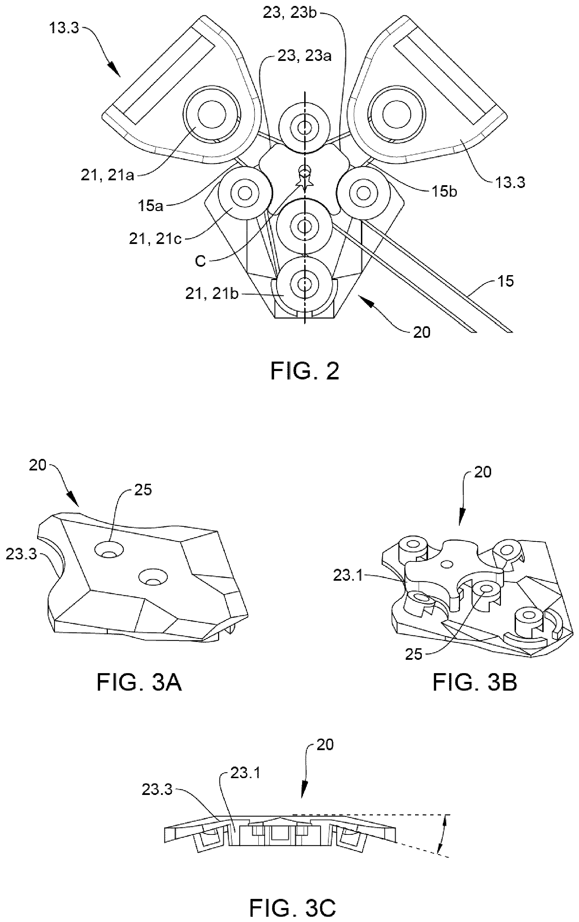

In **Fig. 2** ist eine Anordnung von Zentriereinheit 20 und Kupplungsabschnitten 13.3 relativ zueinander gezeigt, wobei ein Oberteil/Deckel der Zentriereinheit 20 nicht dargestellt ist, um den Blick auf die Umlenkrollen 21 freizugeben. Die Zentriereinheit 20 weist bei diesem Ausführungsbeispiel fünf Umlenkrollen 21 auf, nämlich drei zentrale bzw. mittig angeordnete Umlenkmittel 21b und zwei lateral außenliegende Umlenkmittel 21c, wobei das Zugmittel 15 um alle diese Rollen herum geführt ist, insbesondere derart dass sich eine sternförmige Kraftwirkung radial nach außen auf die Achsen der Umlenkrollen ergibt. Einzige Ausnahme ist die unterste Rolle 21b, welche im Sinne eines Positionsausgleichs vorgesehen ist und zusammen mit der darüber weiter oben angeordneten Rolle S-förmig vom Zugmittel 15 umlaufen wird. An der Rollenachse der letztgenannten Rolle 21b ist eine der beiden Befestigungsachse zum Befestigen der Zentriereinheit 20 an der Rückenschiene 11 vorgesehen. Auch am jeweiligen Kupplungsabschnitt 13.3 ist ein Umlenkmittel 21a vorgesehen, so dass sich eine gute Flaschenzugwirkung ergibt. Die weitere Befestigungsachse ist im zentralen Bereich eines/des Zugkraftzentrums C vorgesehen, und in diesem Bereich ist die Einheit 20 bevorzugt massiv ausgestaltet, insbesondere um eine robuste Druckfläche für den Anschlag 23 für den Schultergurt 13 bereitstellen zu können.

Fig. 2 veranschaulicht dabei auch die Anordnung eines/des Zugkraftzentrums C; die Rollen 21 sind um das Zentrum C herum angeordnet; im Bereich des Zentrums C kann eine Materialverstärkung vorgesehen sein, insbesondere Vollmaterial. Somit kann unter Bezugnahme auf Fig. 2 auch ein Verlauf des Zugmittels durch die Einheit 20 beschrieben werden: im Uhrzeigersinn gesehen verläuft das Zugmittel 15 ausgehend von der Bedieneinheit (nicht dargestellt) zunächst um die Rolle 21b, welche auch eine Befestigungsachse für die Einheit 20 definiert, und daraufhin bei S-förmigem Verlauf um die darunter (caudal) liegende Rolle 21b, dann weiter zur lateral außen (rechts) angeordneten Rolle 21c, welche innenliegend tangiert wird, und dann weiter zur Rolle 21a des rechten Kupplungsstücks 13.3, von dort wieder zurück zur Einheit 20 und die obere (cranial entlang der Sagittalebene angeordnete) Rolle 21b innenliegend tangierend und spiegelsymmetrisch weiter zur Rolle 21a des linken Kupplungsstücks 13.3 und von dort um zur lateral außen (links) angeordneten Rolle 21c, welche ebenfalls innenliegend tangiert wird, und dann wieder zurück zur Bedieneinheit.

In den **Fig. 3A, 3B, 3C** ist ein beispielhafter Aufbau der Einheit 20 veranschaulicht, hier in diesem Beispiel in einstückiger Ausgestaltung (Grundkörper); wahlweise können auch zwei Schalen (Oberteil und Unterteil) vorgesehen sein. Die untere (bei bestimmungsgemäßer Verwendung ventral am Körper anliegend angeordnete) Seite stellt dabei auch die Lager und Achsen für die Umlenkrollen bereit. Der Grundkörper stellt den Anschlag rechts 23a und den Anschlag links 23b sowie eine/die lateral auslaufende Anschlagskontur 23.3 oder Anschlagsflanke (insbesondere kreissegmentförmig) bereit. Die Oberseite liefert beispielsweise lediglich eine Blenden-Funktion oder eine Art Abdeckung zum Schutz vor Staub und zum Einhausen des Zugmittels und der Rollen, jedoch keine stabilitäts- oder kraftflussrelevante Funktion. Insofern kann der Grundkörper der Einheit 20 demnach strenggenommen auch als "einstückig" bzw. "integral" ausgeführt beschrieben werden, insbesondere bei nach innen (in ventraler Ausrichtung) offen angeordneten Rollen.

Bei einer alternativen Ausgestaltung aus zwei Schalen können beide Schalen form-/kraftschlüssig aneinander verrastet werden, insbesondere aufeinandergestellt werden. Beide Schalen stellen den Anschlag rechts 23a und den Anschlag links 23b sowie eine/die lateral auslaufende Anschlagskontur 23.3 oder Anschlagsflanke (insbesondere kreissegmentförmig) bereit, wobei ein/der jeweilige(r) stirnseitige(r) Steg 23.1 oder dergleichen bevorzugt linienförmige Anschlagsfläche bevorzugt nur von derjenigen Schale bereitgestellt wird, welche auch die Rollen abstützt und welche an die Schiene 11 gekoppelt/koppelbar ist; diese Anordnung liefert auch eine besonders hohe Stabilität. Die Oberschale liefert damit beispielsweise lediglich eine Blenden-Funktion oder eine Art Abdeckung zum Schutz vor Staub und zum Einhausen des Zugmittels und der Rollen, jedoch keine stabilitäts- oder kraftflussrelevante Funktion.

Auch aus Fig. **3B** ist ersichtlich, dass wenigstens eine der Befestigungsachsen (25) dabei auch eine Rollenachse für eines der Umlenkmittel bilden kann. Auch dies begünstigt eine stabile verwindungssteife Anordnung und Abstützung und Krafteinleitung.

Aus Fig. **3C** ist der für die optimierte Ausrichtung des Zugmittels 15 vorteilhafte Neigungswinkel der Rollenachsen der lateral außenliegenden Rollen 21c ersichtlich.

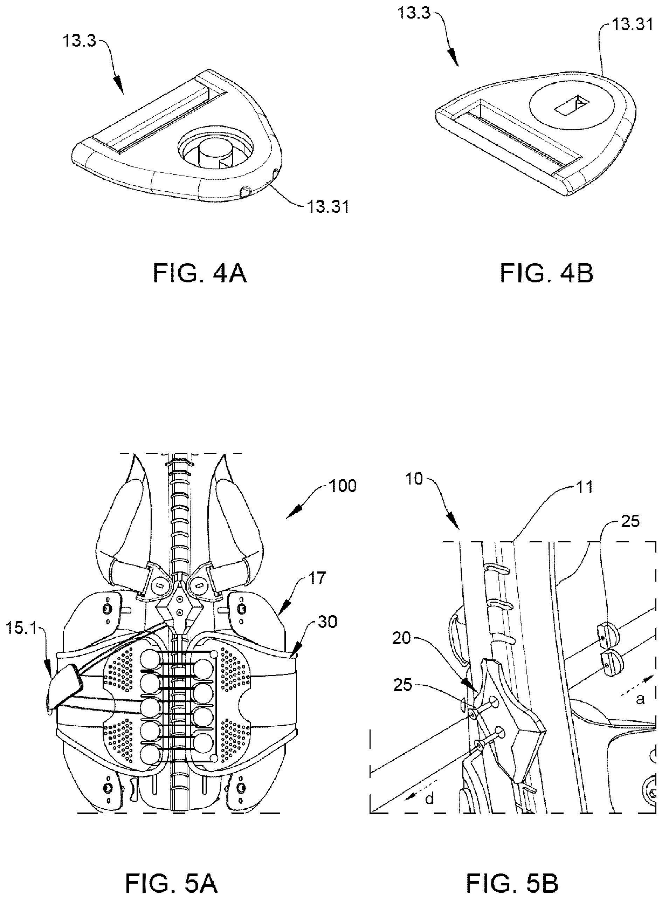

In den **Fig. 4A, 4B** ist der formstabile Kupplungsabschnitt 13.3 des jeweiligen Gurtabschnitts 13a, 13b gezeigt. Der Kupplungsabschnitt 13.3 weist einerseits eine schlitzartige Gurtdurchführung auf (Seite zum Gurt), andererseits ein Lager für die Umlenkrolle 21a (nicht dargestellt) und eine geometrisch korrespondierend zur Zentriereinheit 20 ausgeführte Stirnseite. Der Kupplungsabschnitt 13.3 ist z.B. auch zweiteilig aus zwei Schalen aufgebaut. Wahlweise kann der Kupplungsabschnitt auch einstückig ausgestaltet sein. Ein/der die Zentriereinheit 20 in der Endstellung P kontaktierende Abschnitt (stirnseitige Flanke) des formstabilen Kupplungsabschnitts 13.3 ist mit dem Bezugszeichen 13.31 gekennzeichnet und weist z.B. eine kreissegmentförmige oder elliptische Außenkontur auf.

In **Fig. 5A** ist ein modulares Orthesensystem 100 gezeigt, welches auch einen korsettartigen Hüftgurt 30 umfasst. Die Bedieneinheit 15.1 kann wahlweise am Gurt 30 oder am Seitenteil 17 fixiert werden, wobei am Seitenteil 17 z.B. auch Komponenten eines Klettverschluss vorgesehen sein können. Bevorzugt erfolgt eine Befestigung am Hüftgurt.

In **Fig. 5B** ist im Detail gezeigt, auf welche Weise das Kuppeln der Einheit 20 an die Rückenschiene 11 erfolgen kann. Die entsprechende Befestigungsachse ist hier durch eine gedankliche Linie hervorgehoben. Als Kontermittel sind hier bevorzugt T-Nutensteinen vorgesehen. In einer derartigen direkten Kopplung an die Rückenschiene 11 kann der Kraftfluss nicht nur auf eine vorteilhafte Höhenposition gerichtet sein, sondern auch flächig und ohne Druckspitzen auf den Rücken übertragen werden, insbesondere mittelbar gedämpft auch dank der Rippe 11.1 in der Rückenschiene, welche eine Flexion/Biegung zulassen und damit auch noch eine gute Bewegungsfreiheit der Person 1 zulassen. Gleichzeitig kann auch ein Verrutschen der Zentriereinheit 20 vermieden werden.

Somit kann die Erfindung ein unilateral bedienbares aber symmetrisch wirkendes kinematisches Kraftübertragungskonzept mit Kraftverstärkungsfunktion bereitstellen, welches zudem auch die Handhabung für Laien beträchtlich erleichtert.

Aus der Gesamtoffenbarung geht hervor, dass das Einstellen/Fixieren der Zuglänge des Zugmittels 15 wahlweise auch durch Fixieren der Bedieneinheit 15.1 am Gurt 30 erfolgen kann. Alternativ können entsprechende Flächenabschnitte auch an einem Seitenteil 17 oder einem anderen Gurt bereitgestellt sein.

Der Einfachheit halber werden die an der Schnittstelle zwischen Schiene 11 und Adapter 20 wirkenden Kupplungs-/Befestigungsmittel 25 mit nur einem Bezugszeichen versehen; die Kupplungs-/Befestigungsmittel 25 können sowohl die hier dargestellten Bohrungen/Durchgangslöcher als auch die dafür vorgesehenen Schrauben und Kontermuttern oder T-Nutensteinen aufweisen, oder alternativ gleichwirkende Mittel.

In den Fig. **1A, 1B****,** **5A** sind die Kupplungsabschnitte 13.3 des Schultergurts jeweils in der Endposition P (auf Anschlag gezogen) in Anlage am Adapter 20 gezeigt.

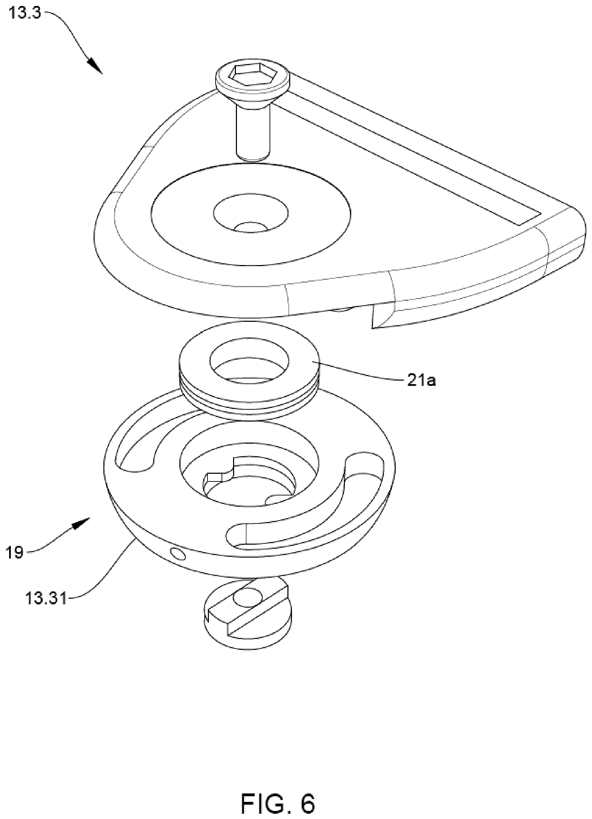

In **Fig. 6** wird eine vorteilhafte Variante eines/des Kupplungsabschnitts 13.3 des jeweiligen Schultergurtabschnitts gezeigt. Diese Variante weist in Abwandlung zu der in den Fig. 4A, 4B gezeigten Variante einen Drehteller 19 mit einem insbesondere durch zwei gegenüberliegende kreissegmentartige Aussparungen vordefinierten Rotationswinkelfreiheitsgrad im Bereich von z.B. ±45° auf (absolut 90°), wobei der Drehteller 19 die entsprechende Umlenkrolle 21a aufnimmt.

Drehteller und Rolle sind zusammen am Grundkörper des Kupplungsabschnitts montiert, insbesondere um dieselbe Achse, z.B. mittels einer Schraubverbindung. Am Grundkörper können Stifte, Vorsprünge oder zylindrische Anschläge oder dergleichen Blockiermittel vorgesehen sein (insbesondere integral), welche in die korrespondierenden Aussparungen im Drehteller eingreifen und dadurch den maximalen Drehwinkel limitieren. Der Drehteller weist dabei bevorzugt auch die zuvor bereits beschriebene stirnseitige Flanke 13.31 auf, so dass der Kupplungsabschnitt mittels der Flanke am Adapter zur Anlage kommen kann; somit wird reibungsarme Drehwinkeltoleranz ermöglicht/sichergestellt. Mittels dieses optionalen Drehtellers kann demnach, falls bei bestimmten Anwendungen erwünscht oder besonders vorteilhaft, eine Reibung auf dem Kraftflusspfad zwischen Schultergurtabschnitt und Bedieneinheit noch weiter vermindert werden, und die Gurte können sich relativ zur Zentriereinheit (Adapter) noch freier bzw. ungehinderter in der optimalen Richtung ausrichten. Abgesehen vom drehwinkeltolerant gekuppelten Drehteller kann der konstruktive Aufbau des Kupplungsabschnitts 13.3 dabei grundsätzlich weitgehend jenem gemäß Fig. 4A, 4B entsprechen, und auch die weiteren zuvor beschriebenen Komponenten können gleichermaßen verwendet werden bzw. für das jeweilige individualisierte Endprodukt personenspezifisch implementiert sein.

### Bezugszeichenliste

- 1: Person
- 10: Rückenorthesevorrichtung
- 11: Rückenschiene
- 11.1: rippenartige Aussparungen
- 11.3: Höhenverstellung, insbesondere Schiene oder Führung
- 13: Schultergurt
- 13a: Schultergurt rechts
- 13b: Schultergurt links
- 13.1: freies Ende des (jeweiligen) Schultergurts
- 13.3: formstabiler Kupplungsabschnitt (insbesondere Verschlusszuglasche)
- 13.31: stirnseitige Flanke
- 15: Zugmittel, insbesondere umlaufend (zumindest im distalen bzw. untenliegenden Abschnitt)
- 15a: Zugmittelabschnitt rechts (bzw. rechte Schulter)
- 15b: Zugmittelabschnitt links (bzw. linke Schulter)
- 15.1: Bedieneinheit
- 17: Seitenteil
- 19: Drehteller
- 20: Zugkraftzentriereinheit bzw. Reklinationsadapter
- 21: Umlenkmittel, insbesondere Rolle
- 21a: Umlenkmittel am formstabilen Kupplungsabschnitt
- 21b: zentral bzw. mittig an der Zugkraftzentriereinheit angeordnetes Umlenkmittel
- 21c: lateral außenliegend an der Zugkraftzentriereinheit angeordnetes Umlenkmittel
- 23: Anschlag für Schultergurt
- 23a, 23b: Anschlag rechts, Anschlag links
- 23.1: stirnseitiger Steg oder dergleichen bevorzugt linienförmige Anschlagsfläche
- 23.3: lateral auslaufende Anschlagskontur oder Anschlagsflanke, insbesondere kreissegmentförmig
- 25: Kupplungs- oder Befestigungsmittel zwischen Zugkraftzentriereinheit und Rückenschiene
- 30: Gurt, insbesondere Hüftgurt
- 100: modulares Orthesensystem mit Rückenorthesevorrichtung
- C: Zugkraftzentrum
- P: Parkposition, formschlüssige Endstellung am Anschlag
- a: anterior, ventral
- d: posterior, dorsal
- u: inferior, distal, caudal
- xz: Sagittalebene

## Patentansprüche

1. Rückenorthesevorrichtung (10), insbesondere eingerichtet zur Reklination des Rückens einer Person (1), aufweisend eine Rückenschiene (11) und wenigstens einen an der Rückenschiene befestigten Schultergurt (13) und für die rechte und linke Schulter der Person jeweils wenigstens einen über den oder im Schultergurt geführten oder am Schultergurt befestigten Zugmittelabschnitt (15a, 15b), wobei diese Zugmittelabschnitte an die Rückenschiene (11) gekoppelt/koppelbar sind und zum Einstellen der vom Schultergurt auf den Rücken ausgeübten Kraft bedienbar sind;
**dadurch gekennzeichnet, dass** die Rückenorthesevorrichtung (10) eine an die Rückenschiene (11) gekoppelten Zugkraftzentriereinheit (20) aufweist, über/durch welche die auf beide Schultern wirkenden Zugmittelabschnitte (15a, 15b) zentral geführt sind und zusammen bedienbar sind.

2. Rückenorthesevorrichtung (10) nach Anspruch 1, wobei die auf beide Schultern wirkenden Zugmittelabschnitte (15a, 15b) durch ein beidseitig wirkendes umlaufendes Zugmittel (15) bereitgestellt sind, welches bevorzugt unilateral an nur einer Seite der Person geführt/führbar und bedienbar ist.

3. Rückenorthesevorrichtung (10) nach dem vorhergehenden Anspruch, wobei das beidseitig wirkende umlaufende Zugmittel (15) aus der Zugkraftzentriereinheit (20) herausgeführt ist, insbesondere mit zwei Strängen, und in einem lateralen und/oder ventralen Bereich der Person handhabbar und bevorzugt auch fixierbar ist, beispielsweise an einem lateral und/oder ventral verlaufenden Klettverschlussabschnitt der Rückenorthesevorrichtung (10) oder eines zusätzlichen Gurts, insbesondere Hüftgurts.

4. Rückenorthesevorrichtung (10) nach einem der Ansprüche 2 oder 3, wobei das Zugmittel (15) in der Zugkraftzentriereinheit (20) um ein Zugkraftzentrum (C) herum geführt ist, wobei die Zugkraftzentriereinheit (20) bevorzugt im Bereich des Zugkraftzentrums an die Rückenschiene (11) gekoppelt ist.

5. Rückenorthesevorrichtung (10) nach einem der vorhergehenden Ansprüche, wobei die Zugkraftzentriereinheit (20) eine Mehrzahl von Umlenkmitteln (21), insbesondere Rollen aufweist, beispielsweise vier oder fünf Umlenkmittel, wobei zumindest lateral außenliegende Umlenkmittel (21c) bevorzugt in einer bezüglich einer/der Frontalebene geneigten Ausrichtung angeordnet sind, insbesondere mit einem Neigungswinkel im Bereich von 3° bis 25°, insbesondere 10° bis 20°.

6. Rückenorthesevorrichtung (10) nach einem der Ansprüche 2 bis 4, wobei die Zugkraftzentriereinheit (20) eine Mehrzahl von Umlenkmitteln (21) aufweist, welche in Flaschenzuganordnung zur Erhöhung der vom Zugmittel auf den Schultergurt ausgeübten Zugkraft vorgesehen ist, insbesondere mit zumindest einer Umlenkung zwischen einem/dem jeweiligen freien Ende (13.1) des Schultergurts (13) und dem/den korrespondierenden Umlenkmittel(n) der Zugkraftzentriereinheit (20), insbesondere mit zumindest einem Umlenkpunkt oder zumindest einer Umlenkachse am jeweiligen freien Ende des Schultergurts.

7. Rückenorthesevorrichtung (10) nach einem der vorhergehenden Ansprüche, wobei die Zugkraftzentriereinheit (20) für den Schultergurt (13, 13a, 13b) eine Endstellung (P) definiert, insbesondere indem für einen jeweiligen Schultergurtabschnitt ein formstabiler Kupplungsabschnitt (13.3) vorgesehen ist, welcher mittels der Zugmittelabschnitte (15a, 15b) formschlüssig in der Endstellung (P) positionierbar ist.

8. Rückenorthesevorrichtung (10) nach dem vorhergehenden Anspruch, wobei der Schultergurt (13) in der Endstellung (P) in einer durch die Zugkraftzentriereinheit (20) vorgegebenen Position angeordnet ist, insbesondere dort einrastet oder dort zumindest formschlüssig positionierbar ist.

9. Rückenorthesevorrichtung (10) nach einem der beiden vorhergehenden Ansprüche, wobei das die Zugmittelabschnitte derart geführt sind, die Zugkraft unabhängig vom Erreichen der Endstellung auf die Schultern aufbringbar ist.

10. Rückenorthesevorrichtung (10) nach einem der vorhergehenden Ansprüche, wobei die Zugkraftzentriereinheit (20) an einer Unterseite Kupplungs- oder Befestigungsmittel (25) für eine Befestigung an der Rückenschiene (11) aufweist, wobei die Position eines/des Befestigungspunktes bevorzugt in Höhenrichtung, insbesondere bei bestimmungsgemäßer Verwendung der Rückenschiene in der Sagittalebene (xz) liegend, einstellbar durch die Rückenschiene (11) vordefinierbar ist, insbesondere mittels einer Schiene oder Führung (11.3) an der Rückenschiene.

11. Rückenorthesevorrichtung (10) nach einem der vorhergehenden Ansprüche, wobei das die Zugmittelabschnitte umlaufend sind und in einer Bedieneinheit (15.1) zusammengeführt sind, welche ausgestaltet ist, beim An-/Ablegen des Schultergurts (13) an der Zugkraftzentriereinheit (20) zu blockieren, und wobei die Zugmittelabschnitte derart abgelängt sind, dass der jeweilige Schultergurtabschnitt bei kumulierter freier Zugweglänge beider Seiten cranial/lateral freilegbar ist.

12. Modulares Orthesensystem (100) mit einer Rückenorthesevorrichtung (10) nach einem der vorhergehenden Ansprüche, wobei das Orthesensystem (100) ferner einen Hüftgurt (30) umfasst, wobei die auf beide Schultern wirkenden Zugmittelabschnitte (15a, 15b) durch ein beidseitig wirkendes umlaufendes Zugmittel (15) bereitgestellt sind, welches bevorzugt in einem lateral und/oder ventral verlaufenden Klettverschlussabschnitt des Hüftgurts fixierbar ist.

13. Verwendung einer an eine Rückenschiene (11) einer Rückenorthesevorrichtung nach einem der Ansprüche 1-11 gekoppelten/koppelbaren Zugkraftzentriereinheit (20) zum zentralen Führen und gemeinsamen Bedienen von wenigstens zwei auf die Schultern einer die Rückenorthese tragenden Person (1) wirkenden Zugmittelabschnitten (15a, 15b), insbesondere im Zusammenhang mit einer Reklination des Rückens der Person, wobei die Zugmittelabschnitte jeweils derart an einen an der Rückenschiene befestigten Schultergurt (13) gekoppelt sind/werden, dass zur Zugkraftaufbringung und zum Einstellen der vom Schultergurt ausgeübten Kraft die Zugmittelabschnitte (15a, 15b) lediglich unilateral zu betätigen sind, insbesondere mittels einer die Zugmittelabschnitte zusammenführenden Bedieneinheit (15.1), wobei die Zugmittelabschnitte bevorzugt durch ein beidseitig wirkendes umlaufend durch die Zugkraftzentriereinheit geführtes und darin um ein Zugkraftzentrum (C) in der Art eines Flaschenzugs umgelenktes Zugmittel (15) bereitgestellt sind, insbesondere in einem modularen Orthesensystem (100) zusammen mit einem Hüftgurt (30).

## Claims

1. A back orthosis device (10), in particular configured to recline the back of a person (1), comprising a back splint (11) and at least one shoulder strap (13) secured to the back splint, and comprising for each the person's right and left shoulders at least one tension member section (15a, 15b) guided over or within the shoulder strap or secured to the shoulder strap, wherein these tension member sections are coupled or couplable to the back splint (11) and can be operated to adjust the force exerted by the shoulder strap on the back;
**characterised in that** the back orthosis device (10) comprises a tension force centring unit (20) coupled to the back splint (11), via which or by means of which the tension member sections (15a, 15b) acting on both shoulders are guided centrally and can be operated together.

2. A back orthosis device (10) according to claim 1, wherein the tension member sections (15a, 15b) acting on both shoulders are provided by a circumferential tensioning means (15) acting on both sides, which is preferably guided/guidable and operable unilaterally on only one side of the person.

3. A back orthosis device (10) according to the preceding claim, wherein the circumferential tensioning means (15) acting on both sides extends from the tension force centring unit (20), in particular extending with two strands, and can be handled and, preferably, also secured in a lateral and/or ventral region of the person, for example to a laterally and/or ventrally extending velcro fastening section of the back orthosis device (10) or of an additional strap, in particular a hip strap.

4. A back orthosis device (10) according to one of claims 2 or 3, wherein the tensioning means (15) is routed within the tension force centring unit (20) around a tension force centre (C), wherein the tension force centring unit (20) is preferably coupled to the back splint (11) in the region of the tension force centre.

5. A back orthosis device (10) according to one of the preceding claims, wherein the tension force centring unit (20) comprises a plurality of deflection means (21), in particular rollers, for example four or five deflection means, wherein at least laterally outermost deflection means (21c) are preferably arranged in an orientation inclined relative to a/the frontal plane, in particular with an angle of inclination in the range of 3° to 25°, in particular 10° to 20°.

6. A back orthosis device (10) according to any one of claims 2 to 4, wherein the tension force centring unit (20) comprises a plurality of deflection means (21), which are arranged in a pulley block configuration to increase the tensile force exerted by the tensioning means on the shoulder strap, in particular with at least one deflection between a/the respective free end (13.1) of the shoulder strap (13) and the corresponding deflection means of the tension force centring unit (20), in particular with at least one deflection point or at least one deflection axis at the respective free end of the shoulder strap.

7. A back orthosis device (10) according to one of the preceding claims, wherein the tension force centring unit (20) defines an end position (P) for the shoulder strap (13, 13a, 13b), in particular by means of a dimensionally stable coupling section (13.3) provided for a respective shoulder strap section, which can be positioned in the end position (P) in form-fit manner by means of the tension member sections (15a, 15b).

8. A back orthosis device (10) according to the preceding claim, wherein the shoulder strap (13) is arranged in the end position (P) in a position predetermined by the tension force centring unit (20), in particular where it engages or can at least be positioned there in form-fit manner.

9. A back orthosis device (10) according to one of the two preceding claims, wherein the tension member sections are guided in such a way that the tensile force can be applied to the shoulders independently of the end position being reached.

10. A back orthosis device (10) according to one of the preceding claims, wherein the tension force centring unit (20) comprises, on a lower side, coupling or fastening means (25) for fastening to the back splint (11), wherein the position of a/the fastening point is preferably pre-definable in the vertical direction by the back splint (11), in particular in the sagittal plane (xz) when the back splint is being used as intended, in particular by means of a rail or guide (11.3) provided on the back splint.

11. Back orthosis device (10) according to one of the preceding claims, wherein the tension member sections are circumferential and are brought together in an operating unit (15.1), which is designed to lock onto the tension force centring unit (20) when the shoulder strap (13) is put on or taken off, and wherein the tension member sections are cut to length such that the respective shoulder strap section can be exposed cranially/laterally when the cumulative free travel length of both sides is taken into account.

12. Modular orthotic system (100) comprising a back orthosis device (10) according to one of the preceding claims, wherein the orthotic system (100) further comprises a hip strap (30), wherein the tension member sections (15a, 15b) acting on both shoulders are provided by a circumferential tensioning means (15) acting on both sides, which can preferably be secured in a velcro fastening section extending laterally and/or ventrally and being part of the hip strap.

13. Use of a tension force centring unit (20) coupled to or couplable to a back splint (11) of a back orthosis device according to any one of the preceding claims 1 to 11, for the central guidance and joint operation of at least two tension member sections (15a, 15b) acting on the shoulders of a person (1) wearing the back orthosis, particularly in connection with a reclination of the person's back, wherein the tension member sections are each coupled to a shoulder strap (13) secured to the back splint in such a way that, for the application of tensile force and for adjusting the force exerted by the shoulder strap, the tension member sections (15a, 15b) need only be actuated unilaterally, in particular by means of an operating unit (15.1) bringing together the tension member sections, wherein the tension member sections are preferably provided by a tensioning means (15) acting on both sides and being guided circumferentially through the tension force centring unit and deflected therein about a tension force centre (C) in the manner of a pulley block, in particular in a modular orthotic system (100) together with a hip strap (30).

## Revendications

1. Dispositif d'orthèse dorsale (10), notamment conçu pour la réclinaison du dos d'une personne (1), comprenant une barre dorsale (11) et au moins une sangle d'épaule (13) fixée à la barre dorsale, ainsi que, pour les épaules droite et gauche de la personne, au moins une section de moyens de traction respectif (15a, 15b) étant guidée respectivement par-dessus ou à l'intérieur de la sangle d'épaule ou étant fixée à la sangle d'épaule, ces sections de moyens de traction étant couplées ou pouvant être couplées à la barre dorsale (11) et pouvant être actionnées pour régler la force exercée par la sangle d'épaule sur le dos ;
**caractérisé en ce que** le dispositif d'orthèse dorsale (10) comporte une unité de centrage de la force de traction (20) couplée à la barre dorsale (11), par l'intermédiaire de laquelle les sections de moyens de traction agissant sur les deux épaules (15a, 15b) agissant sur les deux épaules sont guidées de manière centrale et peuvent être actionnées conjointement.

2. Dispositif d'orthèse dorsale (10) selon la revendication 1, dans lequel les sections de moyens de traction (15a, 15b) agissant sur les deux épaules sont fournies par un moyen de traction périphérique (15) agissant des deux côtés, qui est de préférence guidé et actionnable de manière unilatérale sur un seul côté de la personne.

3. Dispositif d'orthèse dorsale (10) selon la revendication précédente, dans lequel le moyen de traction périphérique (15) agissant des deux côtés sort de l'unité de centrage de la force de traction (20), en particulier avec deux brins, et pouvant être manipulé et, de préférence, également fixé dans une zone latérale et/ou ventrale de la personne, par exemple sur une partie à fermeture velcro s'étendant latéralement et/ou ventralement du dispositif d'orthèse dorsale (10) ou d'une sangle supplémentaire, en particulier une sangle de hanche.

4. Dispositif d'orthèse dorsale (10) selon l'une des revendications 2 ou 3, dans lequel le moyen de traction (15) est guidé dans l'unité de centrage de la force de traction (20) autour d'un centre de force de traction (C), l'unité de centrage de la force de traction (20) étant de préférence couplée à la barre dorsale (11) dans la zone du centre de force de traction.

5. Dispositif d'orthèse dorsale (10) selon l'une des revendications précédentes, dans lequel l'unité de centrage de la force de traction (20) comporte une pluralité de moyens de renvoi (21), notamment des rouleaux, par exemple quatre ou cinq moyens de renvoi, au moins les moyens de renvoi (21c) situés latéralement à l'extérieur étant de préférence disposés selon une orientation inclinée par rapport à un/au plan frontal, notamment avec un angle d'inclinaison compris entre 3° et 25°, en particulier entre 10° et 20°.

6. Dispositif d'orthèse dorsale (10) selon l'une des revendications 2 à 4, dans lequel l'unité de centrage de la force de traction (20) comporte une pluralité de moyens de renvoi (21), qui sont prévus selon une configuration de palan afin d'augmenter la force de traction exercée par le moyen de traction sur la sangle d'épaule, en particulier avec au moins un renvoi entre une/l'extrémité libre respective (13.1) de la sangle d'épaule (13) et le ou les moyens de renvoi correspondants de l'unité de centrage de la force de traction (20), en particulier avec au moins un point de renvoi ou au moins un axe de renvoi au niveau de l'extrémité libre respective de la sangle d'épaule.

7. Dispositif d'orthèse dorsale (10) selon l'une des revendications précédentes, dans lequel l'unité de centrage de la force de traction (20) définit, pour la sangle d'épaule (13, 13a, 13b) une position finale (P), notamment en prévoyant, pour chaque tronçon de sangle d'épaule, un tronçon d'accouplement indéformable (13.3) qui peut être positionné par complémentarité de forme dans la position finale (P) à l'aide des sections de moyens de traction (15a, 15b).

8. Dispositif d'orthèse dorsale (10) selon la revendication précédente, dans lequel la sangle d'épaule (13) est disposée, dans la position finale (P), dans une position prédéfinie par l'unité de centrage de la force de traction (20), en particulier s'y enclenche ou peut au moins y être positionnée par complémentarité de forme.

9. Dispositif d'orthèse dorsale (10) selon l'une des deux revendications précédentes, dans lequel les sections des moyens de traction sont guidés de telle sorte que la force de traction puisse être appliquée sur les épaules indépendamment de l'atteinte de la position finale.

10. Dispositif d'orthèse dorsale (10) selon l'une des revendications précédentes, dans lequel l'unité de centrage de la force de traction (20) comporte, sur une face inférieure, des moyens d'accouplement ou de fixation (25) destinés à la fixation à la barre dorsale (11), la position du/d'un point de fixation pouvant être prédéfinie de préférence dans le sens de la hauteur, en particulier lorsque la barre dorsale est utilisée conformément à sa destination, dans le plan sagittal (xz), et peut être prédéfinie d'une manière ajustable par la barre dorsale (11), notamment au moyen d'un rail ou d'un guide (11.3) sur la barre dorsale.

11. Dispositif d'orthèse dorsale (10) selon l'une des revendications précédentes, dans lequel les sections de moyens de traction sont périphériques et sont réunies dans une unité de manipulation (15.1) qui est conçue pour se bloquer au niveau de l'unité de centrage de la force de traction (20) lors de la mise en place ou du retrait de la sangle d'épaule (13), et dans lequel les sections de moyens de traction sont coupées à longueur de telle sorte que le tronçon de sangle d'épaule correspondant puisse être dégagé en direction crânienne/latérale lorsque la longueur cumulée de la course libre des deux côtés est atteinte.

12. Système d'orthèse modulaire (100) comprenant un dispositif d'orthèse dorsale (10) selon l'une des revendications précédentes, le système d'orthèse (100) comprenant en outre une sangle de hanche (30), dans lequel les sections de moyens de traction agissant sur les deux épaules (15a, 15b) sont fournies par un moyen de traction périphérique (15) agissant des deux côtés, qui peut de préférence être fixé dans une partie de fermeture velcro de la sangle de hanches s'étendant latéralement et/ou ventralement.

13. Utilisation d'une unité de centrage de la force de traction (20), couplée ou pouvant être couplée à une barre dorsale (11) d'un dispositif d'orthèse dorsale selon l'une des revendications précédentes 1 à 11, pour le guidage centralisé et la manipulation conjointe d'au moins deux sections de moyens de traction (15a, 15b) agissant sur les épaules d'une personne (1) portant l'orthèse dorsale, notamment dans le cadre d'une réclinaison du dos de la personne, les tronçons de moyens de traction étant respectivement couplés à une sangle d'épaule (13) fixée à la barre dorsale de telle sorte que, pour appliquer la force de traction et régler la force exercée par la sangle d'épaule, les tronçons de moyens de traction (15a, 15b) ne doivent être actionnées que d'un seul côté, notamment au moyen d'une unité de manipulation (15.1) réunissant les sections de moyens de traction, les sections de moyens de traction étant de préférence constituées d'un moyen de traction (15) agissant des deux côtés, guidé d'une manière périphérique à travers l'unité de centrage de la force de traction et y étant dévié autour d'un centre de force de traction (C) à la manière d'un palan, en particulier dans un système d'orthèse modulaire (100) associé à une sangle de hanche (30).
